# EUROPEAN PATENT APPLICATION

(11) **EP 1 444 998 A1**
(43) Date of publication of application: **11.08.2004**
(21) Application number: 03002536.5
(22) Date of filing: 05.02.2003
(51) Int. Cl.: A61M 1/34, B01D 35/30

(54) **Bio-liquid treatment device**

(71) Applicant: B. Braun Medizintechnologie GmbH, 34212 Melsungen (DE)
(72) Inventor: Lüning, Rudolph, 37083 Göttingen (DE); Purcell, Lawrence, Toronto M9N3H5 Ontario (CA); Geronemous M.D., Robert, Lauderdale Lakes Florida 33313 (US)
(74) Representative: Herzog, Markus, Dipl.-Phys. Dr.

(57) **Abstract**

The invention relates to a bio-liquid treatment device (10) comprising at least two bio-liquid treatment modules (12, 14, 16) containing a bio-liquid treatment medium (12g, 14g, 16g) , the bio-liquid treatment media of at least two bio-liquid treatment modules being different from each other. A series of such bio-liquid treatment modules (12, 14, 16) may provide a bio-liquid treatment device (10) which can provide an enhanced nephrologic therapy, in particular hemodiafiltration.

## Description

The invention relates to a bio-liquid treatment device comprising at least two bio-liquid treatment modules containing a bio-liquid treatment medium.

In the context of the present application, the term "bio-liquid" refers, for example, to blood, blood plasma, urine or any other body liquid. For facilitating the understanding, the bio-liquid treatment device will be described and discussed in the following, mostly with reference to a nephrologic device, in particular, provision of a hemodiafiltration device, as an example. It should be noted, however, that the bio-liquid treatment device according to the present invention may be also used for a plurality of other purposes.

A known bio-liquid treatment device is, for example, sold under the trademark TriEx-1 by Extracorporeal S.A., Belgium. This known device comprises three identical bundles of hollow fibers arranged in three chambers of the device. In use, the blood travels through the three chambers simultaneously, i.e. in parallel, from the top of the device to the bottom thereof. The dialysate fluid in contrast travels through the chambers sequentially.

Another known device is sold under the trademark MD Diafilter by Nephros, Inc., USA. The construction of the MD Diafilter is similar to a typical nephrologic device using a single bundle of hollow fibers. A ringshaped groove in the potting compound at one end and a two-compartment header cap are used to separate the fiber bundle into two discrete but serially connected blood paths. The dialysate fluid is flowed into the device at one end and out at the other end, the dialysate fluid flows co-current to the blood flow in a first circuit and counter-current to the blood flow in the second circuit. Expensive sterile physiological replacement fluid is added to the blood after the first circuit. Further an expensive controller is necessary to control the flows.

In contrast to the known bio-liquid treatment devices, the present invention provides a bio-liquid treatment device which has a much more universal range of possible applications as the bio-liquid treatment media of at least two bio-liquid treatment modules are different from each other. Further it eliminates the necessity for expensive replacement solutions and spezialized dedicated equipment for the provision of H.D.F. (**h**emo**d**ia**f**iltration).

At least two bio-liquid treatment modules of the bio-liquid treatment device according to the present invention may comprise at least one bio-liquid inlet and at least one bio-liquid outlet, respectively, at least two of the bio-liquid treatment modules being connected in series with respect to the flow of bio-liquid defined by said bio-liquid inlets and said bio-liquid outlets.

Moreover, at least one bio-liquid treatment module may comprise a bio-liquid compartment and a working liquid compartment, said working liquid compartment comprising a working liquid inlet and a working liquid outlet, said bio-liquid compartment and said working liquid compartment being separated from each other by a separating medium separating the bio-liquid from the working liquid but allowing the transfer of predetermined substances. By this, the bio-liquid treatment module may be used as a filter filtering, for example, small and mid-sized molecules from the bio-liquid, e.g. blood, in particular urea, creatinine, vitamine B12 or the like. A further example is that a device may be applied to plasma separation/treatment.

The separating medium may be chosen in accordance with the specific needs and requirements and may be, for example, a membrane and/or activated carbon and/or adsorption/absorption particles and/or ion exchange media and/or adsorption membrane.

The adsorption/absorption particles may, for example, be formed as fibers, pellets, e.g. made from resin, and may be coated with enzymes and/or antigens or may have ion-exchange properties.

The separating membranes, e.g. permeable membranes or semi-permeable membranes, may be formed by the wall material of a plurality of hollow fibers. In this case the bio-liquid compartment is formed by the inside of the hollow fibers and the working liquid compartment is formed by the outside of the hollow fibers or vice versa.

The specific properties of the membranes may be chosen by a corresponding choice of the hollow fiber wall material. Typical materials which may be used are synthetic and/or natural material, e.g. polysulphone, polymethyl methacrylate, polyamide, polyethersulphone, polyetherimide, cuprophane, hemophane. Additional to the choice of material, the pore structure, porosity, sieving curve properzies can be selected, e.g. Low Flux, Mid Flux, High Flux, µ Filtration.

In addition to the separating medium there may be provided in the working liquid compartment of at least one bio-liquid treatment module a bio-liquid treatment medium, e.g. a granulate of activated carbon, ion exchange resin or the like.

If the working liquid flows through the working liquid compartment in co-flow or counter-flow with respect to the flow of bio-liquid, a filtering and washing operation of bio-liquid may be realized. It is, however, also conceivable that the working liquid inlet of at least one bio-liquid treatment module is closed. In this case, a treated pure ultra-filtering operation is achieved, where the treated Ultrafiltrate may be returned directly to the bio-liquid circuit.

In a preferred embodiment of the present invention the bio-liquid treatment compartments may be connected in series with respect to the flow of working liquid, the working liquid flowing in couter-flow with respect to the flow of bio-liquid. This ensures an enhanced filtering and washing effect as the cleanest working liquid is introduced into the last (with respect to the flow of bio-liquid) bio-liquid treatment module in which it is "in contact" with already highly purified bio-liquid having only residual concentrations of the substances to be extracted from the bio-liquid. As the clean working liquid has a neglectable concentration of these substances, also in the last bio-liquid treatment module a further purification of the bio-liquid may be achieved.

The above-described cleaning and washing operation may further be enhanced by providing a series of at least three bio-liquid treatment modules, an intermediate bio-liquid treatment module of this series of bio-liquid treatment modules having a higher flow resistance than a preceding bio-liquid treatment module and a succeeding bio-liquid treatment module. The terms "preceding" and "succeeding" used in this context refer to the flow of bio-liquid. The higher flow resistance in the intermediate bio-liquid treatment module enhances the convection and consequent expelling of extra undesired substances, thus leading to a highly efficient purification system. The higher flow resistance in the intermediate bio-liquid treatment module supports the expelling of undesired substances in the preceding bio-liquid treatment module, thus leading to a highly efficient pre-purification in this preceding module. The higher flow resistance in the intermediate bio-liquid treatment module may be achieved by use of fibers of lower inner diameter and/or fiber wall materials of different flux, fibers of different wall thicknesses and/or a lower number of fibers and/or fibers of longer length. It is, however, also conceivable to use a conventional flow restrictor in or as the intermediate bio-liquid treatment module. The final module is used for re-hydration, electrolyte amelioration and assisting with clearances. The third module may have fibers of larger diameter to the other modules further reducing the overall bio-liquid pressure drop. This module may have an overall lower surface area reducing the amount of separating material required, as filtration is more easily facilitated from aqueous solution.

Such a multi-module bio-liquid treatment device may be used, for example, as a dialyzing device, in particular a hemodiafiltration device, providing an enhanced clearance of small, mid-sized and large-sized molecules, e.g. providing at least a clearance of 450 ml/minute for urea, 400 ml/minute for creatinine, 290 ml/minute for vitamine B12 measured at a blood flow of 500 ml/minute, a dialysate flow of 800 ml/minute, and an ultra-filtration rate of 0 ml/minute in an aqueous solution with at least 3 sqm surface area. But also an enhanced clearance of larger molecules in a similar manner to that of the natural kidney may be achieved.

Due to its modular setup, the bio-liquid treatment device according to the present invention provides a high degree of flexibility with respect to the specific requirements of the physician/clinician. As other known bio-liquid treatment devices it may be used as a disposable unit that may be connected to a clinical dialyzing equipment. In contrast to the known bio-liquid treatment devices, however, it may be used with any standard High-Flux dialyzing machine which together provides a hemodiafiltration therapy. I.e. (and this should be emphasized) a hemodiafiltration may be performed without the need for a specific high-level hemodiafiltration machine and/or controlling device. Moreover, using the bio-liquid treatment device according to the present invention it is not necessary to provide expensive infusion solutions as this is the case with the known hemodiafiltration devices, all of which contribute to raising the known safety threshold of hemodialysis.

The present invention allows for the provision of hemodiafiltration within the pre-existing standard safety limits of hemodialysis.

In summary, the present invention provides a superior dialyzing device which allows for the shortening of the time on dialysis for a given prescription and/or to enhance the degree of bio-liquid purification within a given period of time. This means that the amount of time on "daily dialysis" programs may be shortened and that the superior dialysis therapy of "nocturnal" programs may be achieved due to the enhanced clearance of small mid and large sized molecules.

In particular, the above-described three-module device provides a superior complex therapy of hemodiafiltration. The first module acts like Glomerulus in the natural kidney where large quantities of ultra-filtrate are produced. The second module acts like the Proximal Convoluted Tubule and Loop of Henley and the final module acts in a similar, manner to the Distal . Convoluted Tubule where appropriate amounts of salt and water are reabsorbed into the blood.

By choosing the overall length of the hollow fibers in the preceding compartment, the intermediate compartment and the succeeding compartment to between about 50 mm and about 230 mm any detrimental effects and any harm to the patient due to excessive hematocrite values can be avoided. Not only due to this fact the bio-liquid treatment device according to the present invention is highly bio-compatible.

As described above, the bio-liquid treatment modules may be combined into units according to the specific requirements of the physician/clinician, these units may be connected to any standard dialysis machine. To this end, for example, at least one bio-liquid treatment module may have a housing separate from the housing of the other bio-liquid treatment modules. It is, however, also conceivable that at least two bio-liquid treatment modules share a common housing. The bio-liquid treatment modules contained in one and the same housing may be arranged in parallel or in series with respect to the flow of the bio-liquid and/or the working liquid.

Two or more modules may be aligned and conjoined in one and the same housing such that the separating walls separating the modules from each other are concentric, co/triaxial, and the like with respect to each other. It is, however, also possible that two or more modules are aligned and conjoined such that the surrounding wall of the center module is essentially cylindrical with one or more D-shapes along its sides. Moreover, two or more modules may be aligned and conjoined such that each module resembles a longitudinal oval. Appropriate connections may be provided in each case in order to articulate the desired flows of bio-liquid and working liquid.

If the housing has an overall longitudinal shape, the outer contour of which is substantially constant over the entire length of the housing, a space-saving and compact side-by-side arrangement of a plurality of housings may be provided. In addition, the outer surface of the cross-section of the housing may have a substantially polygonal, e.g. hexagonal, pentagonal, rectangular, quadratic, triangular, ..., or an at least partially curved, e.g. elliptical, circular, concave, convex (for example half- or quarter-moon shaped) contour. The internal surface may have the same or a different shape, e.g. the outer surface of the housing may have a hexagonal shape, whereas the internal surface may have a circular shape.

In order to facilitate a combination of a plurality of housings to a bio-liquid device unit it is suggested that at least two housings are provided with an interlocking connection. To this end, a housing may comprise at least one of a male connecting structure and a female connecting structure. For example, the housing may have a female connecting structure that may be connected to a corresponding male connecting structure of another housing or of another element to be connected to the housing, e.g. a flexible tube or hose or the like. It is, however, also conceivable to provide both said housing, on the one hand, and said other housing or said other element, on the other hand, with female connecting structures and to furthermore provide an additional connecting piece having two male connecting structures mating with the female connecting structures.

To facilitate the connecting operation of two adjacent housings at least one pair of mating connecting structures may be formed as a slide and lock connection. For example, the male connecting structure may be formed by a dovetail-like projection and the female connecting structure may be formed by dovetail-like recess. Furthermore, a spring-biased ball engaging in a corresponding recess may complete the slide and lock connection.

The at least one connecting structure may have at least one through-hole leading to the interior of the housing. Preferably two through-holes are provided, e.g. one for the transfer of bio-liquid and one for the transfer of working liquid. However, it is also possible to provide two through-holes for the transfer of bio-liquid and/or two through-holes for the transfer of working liquid and/or three through-holes for the transfer of bio-liquid and working liquid (ultra-filtrate, plasma). Each of the through-holes may be surrounded by a sealing ring in order to prevent undesired leakage.

In particular in the case of a bundle of hollow fibers contained in a housing it is preferable to provide a ring chamber in at least one longitudinal end section of the housing. In a first longitudinal end section of the housing which is equipped with the working-liquid inlet, this ring chamber serves as a distributing chamber ensuring a substantially homogeneous distribution of the working-liquid over all hollow fibers of the fiber bundle, and in the respective other longitudinal end section, this ring chamber serves as a collecting ring chamber substantially homogeneously collecting the working-liquid from all hollow fibers of the fiber bundle. The dimension of the ring chamber taken in the longitudinal direction of the housing may amount to between about 0.1 mm and about 3.0 mm. This distribution ring may also be formed by optimization of the fiber bundle volume and reduction of the cross-sectional area at at least one longitudinal end without mechanical' alterations to the housing.

In addition, the ring chamber may be provided by increasing the inner diameter of the housing in at least one longitudinal end section. The outer diameter of the housing may remain essentially constant in order to ensure a compact side-by-side arrangement of a plurality of housings. This requires that the wall thickness of an outer wall of the housing may be reduced in at least one longitudinal end section of the housing. In contrast, it is, however, also conceivable to reduce the cross-section of the envelope surface of the bundle of hollow fibers in at least one longitudinal end section of the housing. In this context, the term "envelope surface" refers to a theoretical surface surrounding the bundle of fibers. Unlike conventional filters, the invention requires that this envelope surface be reduced.

It should be noted that the bio-liquid treatment device according to the present inverntion may not only be used for dialysis. It may furthermore be used for the therapy of Apheresis by cascading appropriate membranes in one disposable device and optionally incorporating appropriate adsorptive media in either one of the modules or within a separate module. Moreover, the device according to the present invention may be used for the H.E.L.P. therapy (**H**eparin induced **E**xtracorporal **L**ipid (LDL) **P**recipitation), the MARS therapy (**M**olecular **A**dorbent **R**ecirculating **S**ystem) and the CRRT therapy (**C**ontinous **R**enal **R**eplacement **T**herapy) by using an appropriate selection of bio-liquid treatment modules.

Further advantages of the bio-liquid treatment device according to the present invention are:
- A reduced amount of replacement solution is required for CRRT therapy.
- The provision of a superior dialysis is facilitated in one disposable device without the need for scientific equipment of replacement solutions.
- Due to the modular approach, unique designer device is possible by mix/matching a variety of modules to more faithfully fulfill the prescription.
- The device provides an ultimate removal of small molecule toxins from the body.
- The device and the resulting therapy improve the patient's sense of well-being over existing systems.
- The device increases clearance of larger molecules over existing methods.
- The device provides for the therapy of Hemodiafiltration in one inexpensive, disposable device that may be used in conjunction with any existing high-flux hemodialysis machine. Consequently dedicated "high-tech" H.D.F. equipment or spezialized control modules which are attached to existing hemodialysis machines are not required.
- The device does not require the use of expensive sterile replacement solutions.
- The therapy of Hemodiafiltration may be conducted within 'the standard safety limits of normal hemodialysis.
- The device may be used for AMD (**A**dvanced **M**acular **D**egeneration).

In the following, the present invention will be described in more detail with reference to the accompanying drawings, in which:
- Fig. 1: is a schematic view of a first embodiment of the present invention, in particular a hemodiafiltration device;
- Fig. 2: is a detailed sectional view of a one-module housing;
- Fig. 3: is a top sectional view of a side-by-side arrangement of a three-housing unit;
- Fig. 4a to 4f: are schematic views of multi-module housings and
- Fig. 5 to 7: are schematic views of further embodiments.

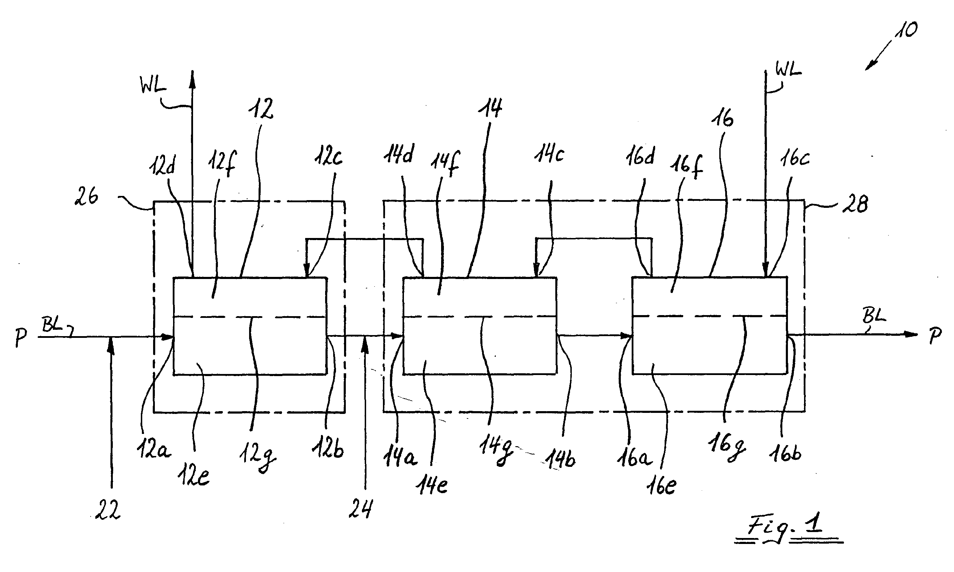

In Fig. 1 a dialysis/filtration/hemodiafiltration device 10 is shown as a first embodiment of the present invention. It comprises three bio-liquid treatment modules 12, 14 and 16 each having a bio-liquid inlet 12a, 14a, 16a, a bio-liquid outlet 12b, 14b, 16b, a working liquid inlet 12c, 14c, 16c and a working liquid outlet 12d, 14, 16d. The three modules 12, 14 and 16 are connected in series with respect to the flow of bio-liquid BL, for example blood, as well as with respect to the flow of working liquid WL. The flows of bio-liquid BL and working liquid WL being in counter-flow condition with respect to each other.

Moreover, each of the modules comprises a bio-liquid compartment 12e, 14e, 16e connected to the respective bio-liquid inlet 12a, 14a, 16a and the respective bio-liquid outlet 12b, 14b, 16b as well as a working liquid compartment 12f, 15, 16f connected to the respective working liquid inlet 12c, 14c, 1 6c and the respective working liquid outlet 12d, 14d, 16d. The respective bio-liquid compartment 12e, 14e, 16e and the respective working liquid compartment 12f, 14f, 16f are separated from each other by a separating medium 12g, 14g, 16g separating the bio-liquid BL and the working liquid WL from each other but allowing the transfer of predetermined substances, e.g. urea or other uremic toxins.

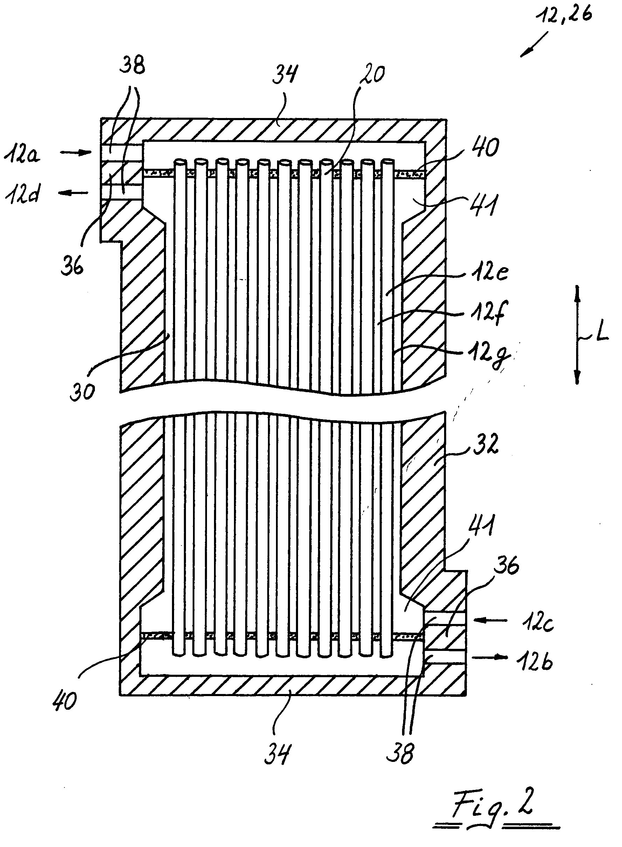

The separating media 12g, 14g, 16g may, e.g. be formed by semi-permeable membranes, the membrane properties of which determine the kind and the size of substances which may pass through the membrane. The membranes may, for example, be formed by the wall material of a bundle of hollow fibers 20 (see Fig. 2).

In order to obtain a purification of the bio-liquid, e.g. blood, there must also be a concentration differential of the substances to be removed from the bio-liquid between the concentration thereof in the bio-liquid chamber 1 2e, 14e, 16e and the concentration thereof in the working liquid chamber 12f, 14f, 16f. As it is desired to obtain a decrease of the concentration of these substances from the bio-liquid coming from the patient P and led into in the first module 12 at 12a to the bio-liquid led out of the last module 16 at 1 6b and going to the patient P, the working liquid WL is in counter-flow with respect to the flow of the bio-liquid BL. Thus, fresh and clean working liquid WL is led into the last module 16, thus ensuring that also in this module there is a considerable concentration differential of the substances to be removed between the bio-liquid compartment 1 6e and the working liquid compartment 16f.

The substances removed from the bio-liquid BL in the last module 16 are washed by the flow of the working liquid WL to module 14 and finally to module 12. However, as the bio-liquid contained in these two modules has a higher concentration of these substances there still is a sufficient concentration differential between the respective bio-liquid compartments 12e, 14e and working liquid compartments 12f, 14f. Thus, the counter-flow of bio-liquid BL and working liquid WL over the series arrangement of all three modules 12, 14, 16 is not detrimental to the purification effect. In contrast, in this manner the best purification of the bio-liquid BL is achieved.

A further enhancement of the purification is obtained by using the intermediate module 14 as a flow restrictor for the flow of bio-liquid BL, thus increasing the bio-liquid pressure in the preceding module 12. Due to this higher pressure prevailing in the bio-liquid compartment 12e of the first module 12 the convective contribution of the transfer of the substances to be removed through the membrane 12g is increased, with consequent enhanced removal of mid and large sized molecules.

The flow restriction property of module 14 may, for example, be obtained by using hollow fibers 20 of a lower inner diameter. The hollow fibers used in modules 12 and 16 may be the same, may in particular have the same inner diameter, length and wall material. It is, however, also conceivable to use hollow fibers 20 of different length and diameter and even different materials.

Furthermore, it should be noted that a replacement solution may be added to the bio-liquid BL either at 22 or 24, i.e. either in pre-stage or in mid-stage.

It is furthermore indicated in Fig. 1 in dashed-dotted lines that the modules 12, 14, 16 may be either contained in separate housings or in common housings. In the embodiment shown in Fig. 1 module 12 is contained in a separate housing 26 and modules 14 and 16 are contained in a common housing 28. It is, however, also conceivable that each of the modules 12, 14 and 16 be contained in a separate housing or that all three modules are contained in one and the same housing.

The separating media may be constructed from different materials which contribute diffrent characteristics, e.g. high, mid or low flux membranes manufactured from natural or synthetic materials.

In Fig. 2 the construction of the housing 26 containing module 12 is shown in more detail.

An inner chamber 30 of the housing 26 is confined by an outer wall 32 and an end caps 34. In the chamber 30 a bundle of hollow fibers 20 is arranged, the inside of these hollow fibers 20 commonly forming the bio-liquid compartment 12e, the outside of the hollow fibers 20 forming the working liquid compartment 12f and the wall material of the fibers 20 forming the semi-permeable membrane 12g. Adjacent to the end caps 34 there are provided connecting structures 36 each having two through-holes 38 forming the bio-liquid inlet 12a, the bio-liquid outlet 12b, the working liquid inlet 12c, and the working liquid outlet 12d.

In a region between the two through-holes 38 allocated to one and the same connecting structure 36 there is provided a layer of sealing material 40 extending essentially parallel with respect to the adjacent end caps 34. In this sealing material 40 the longitudinal end sections of the hollow fibers 20 are embedded. Thus, it is ensured that only bio-liquid led into chamber 30 via the bio-liquid inlet 12a enters into the inside of the hollow fibers 20 and that only purified bio-liquid coming from the inside of the hollow fibers 20 is led out of chamber 30 via bio-liquid outlet 12b. And it is furthermore ensured that only working liquid introduced into chamber 30 via working liquid inlet 12c is surrounding the fibers 20 and is leaving chamber 30 via working liquid outlet 12d.

By reducing of the thickness of the outer wall 32 a ring chamber 41 is formed in the longitudinal end sections of the housing 26. This ring chamber 41 ensures a homogeneous distribution and a homogeneous collection, respectively, of the working-liquid WL over all hollow fibers 20. In addition also the diameter of the hollw fibers 20 may be reduced in the longitudinal end sections of the housing 26 in order to increase the volume of said ring chamber 41.

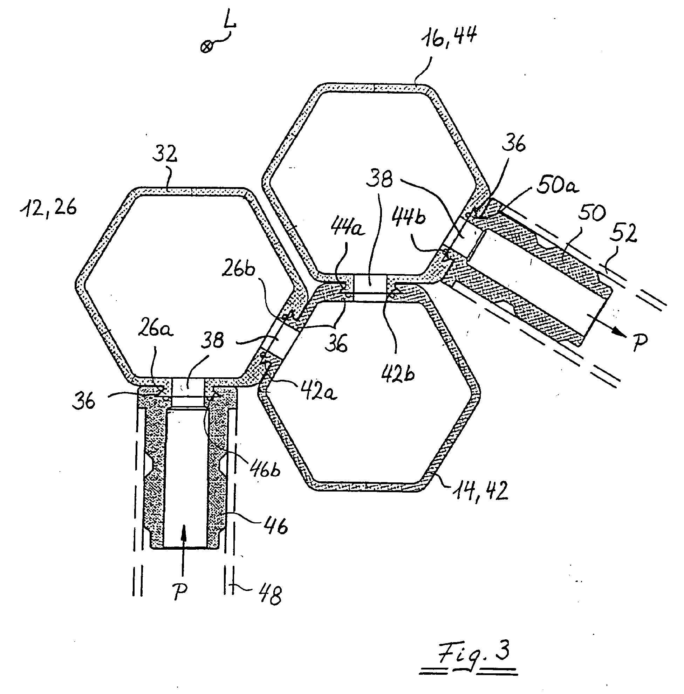

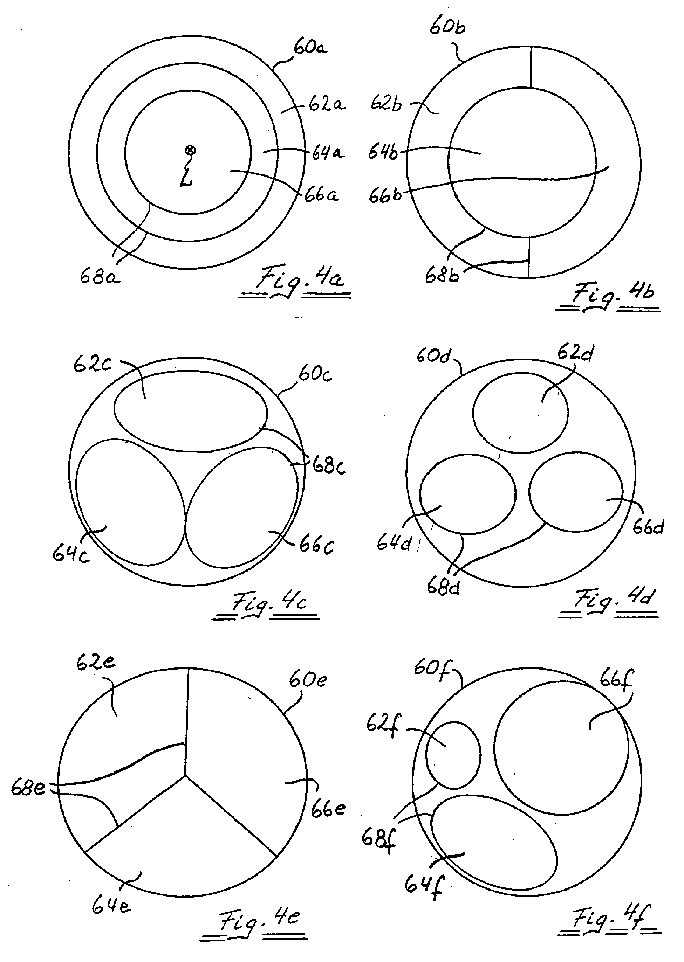

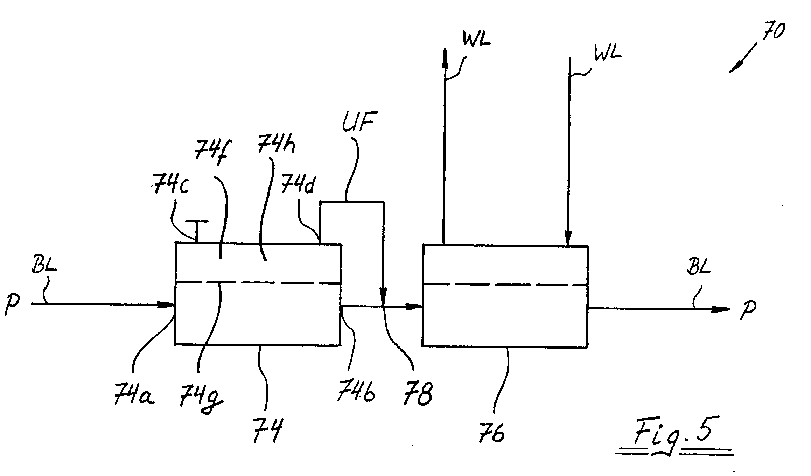

The connecting structures 36 may, for example, have a dovetail-like constrution as is shown in Fig. 3. The embodiment shown in this Figure differs from the embodiment shown in Fig. 1 only insofar as also modules 14 and 16 are contained in separate housings 42 and 44. Furthermore, for facilitating the illustration the connecting structures 36 are shown as being arranged in one and the same plane orthogonal with respect to the longitudinal axis L of the housings 26, 42 and 44.

Each of the housings 26, 42 and 44 has a dovetail-like projection 26a, 42a and 44a and a dovetail-like recess 26b, 42b and 44b mating with each other. A corresponding dovetail-like recess 46b is also formed in a connecting piece 46 of a flexible tube or hose 48 coming from the patient P, and a corresponding dovetail-like projection 50a is provided on a connecting piece 50 of a flexible tube or hose 52 leading to the patient P.

In this way the connections between connecting piece 46 and housing 26, between housing 26 and housing 42, between housing 42 and housing 44 and between housing 44 and connecting piece 50 are formed as slide-and-lock connections, the locking function being realized, for example, by a spring-biased ball (not shown) enganging in a corresponding groove or recess (also not shown). As may easily be seen from Fig. 3 this allows a compact side-by-side arrangement of the housings 26, 42 and 44, and this irrespective of the particular shape of the outer wall of these housings, e.g. wall 32 of housing 26. Although a hexagonal shape of the outer wall of the housings 26, 42 and 44 is shown in Fig. 3, other shapes are also conceivable, e.g. a circular shape.

It is easily understood that on the basis of this modular setup which heralds a new era of "designer devices" that a great variety of different bio-liquid treatment devices may be realized taking the specific needs and requirements of the physicians/clinicians more fully into account.

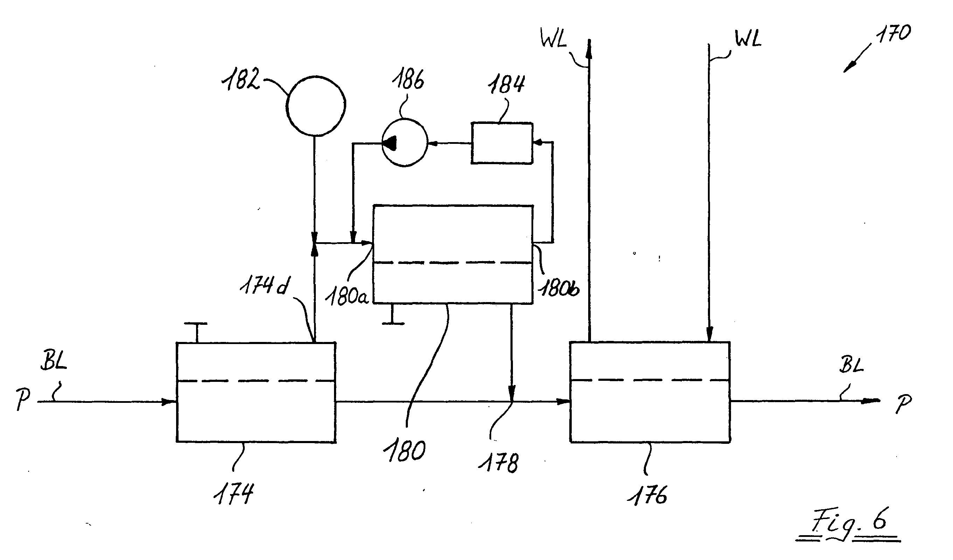

With reference to Figs. 4a to 4f it should be noted that the housings 60a to 60f may also contain a plurality of bio-liquid treatment modules 62a to 62f, 64a to 64f, 66a to 66f which are in side-by-side arrangement to each other parallel to the longitudinal axis L of the housings 60a to 60f and are separated from each other by separating walls 68a to 68f.

For further illustration of the invention in the following additional embodiments of bio-liquid treatment devices shall be discussed with reference to Figs. 5 to 7:

The dialysis device 70 shown in Fig. 5 comprises two modules 74 and 76 that are connected in series with respect to the flow of bio-liquid BL. The first bio-liquid treatment module 74 is used as a separator and an adsorption module, whereas the second bio-liquid treatment module 76 is used as a standard dialyzing module.

To this end, a bundle of hollow fibers 20 forming a membrane 74g is contained in the first module 74, the space 74f surrounding the fibers being filled with activated carbon acting as adsorption medium 74h. By this module the bio-liquid led into the module via bio-liquid inlet 74a is split up into two sections, namely purified bio-liquid leaving the module 74 via bio-liquid outlet 74b and an ultra-filtrate and/or plasma filtrate purified by the activated carbon leaving the module via outlet 74d, the corresponding inlet 74c being closed. The so purified filtrate is returned to the flow of bio-liquid at 78, i.e. before the bio-liquid is led into the second module 76.

The bio-liquid treatment module 76 essentially corresponds to one of the bio-liquid treatment modules 12, 14 and 16 described above.

The embodiment shown in Fig. 6 essentially corresponds to that shown in Fig. 5. Therefore, analogous parts are designated by the same reference numerals as in Fig. 5, however, increased by 100. Moreover, the embodiment according to Fig. 6 will in the following be explained only insofar as it differs from the embodiment according to Fig. 5.

The bio-liquid treatment device 170 differs from the bio-liquid treatment device 70 shown in Fig. 5 by providing an additional treatment of the ultra-filtrate and/or plasma filtrate leaving the first module 174 via outlet 174d before it is returned to the flow of bio-liquid before entering dialyzing module 176.

In particular, the ultra-filtrate and/or plasma filtrate is introduced into another bio-liquid treatment module 180 after addition of a precipitating agent supplied from a corresponding reservoir 182. This precipitating agent serves to precipitate predetermined molecules from the ultra-filtrate. From the outlet 180b the so purified ultra-filtrate is recycled via a reservoir 184 and a pump 186 to the inlet 180a.

In this way, the bio-liquid treatment device 170 allows to perform a therapy which is rather similar to the HELP therapy.

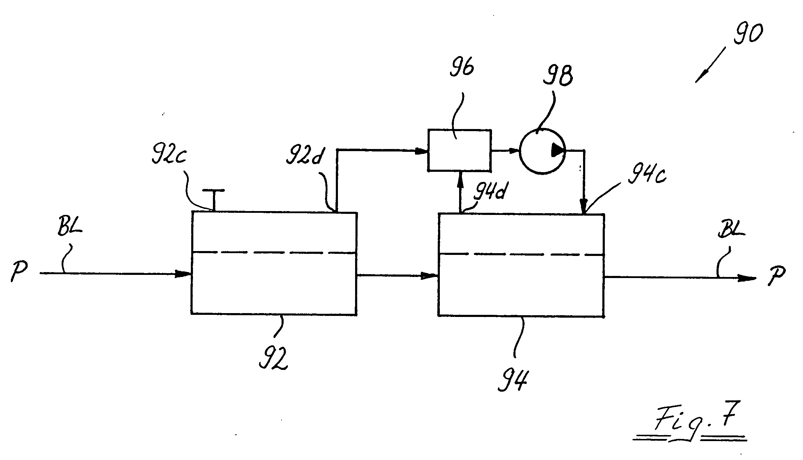

In Fig. 7 a bio-liquid treatment device 80 is shown which may be used for plasma exchange in a cascade filtration.

A first bio-liquid treatment module 92 may, for example, comprise a membrane with a 50k Dalton cutoff, whereas the second module 94 may comprise a membrane with a 40k Dalton cutoff. The two modules 92 and 94 being connected in series with respect to the flow of bio-liquid.

The filtrate leaving the first module 92 via outlet 92d (the corresponding inlet 92c being closed) is led to a reservoir 96 to which also the outlet 94d of the second module 94 is connected. From this reservoir 96 the filtrate is led to the inlet 94c of the second module via a pump 98.

The invention relates to a bio-liquid treatment device (10) comprising at least two bio-liquid treatment modules (12, 14, 16) containing a bio-liquid treatment medium (12g, 14g, 16g) , the bio-liquid treatment media of at least two bio-liquid treatment modules being different from each other. A series of such bio-liquid treatment modules (12, 14, 16) may provide a bio-liquid treatment device (10) which can provide an enhanced nephrologic therapy, in particular hemodiafiltration.

## Claims

1. Bio-liquid treatment device (10) comprising at least two bio-liquid treatment modules (12, 14, 16) containing a bio-liquid treatment medium (12g, 14g, 16g) , the bio-liquid treatment media of at least two bio-liquid treatment modules being different from each other.

2. Bio-liquid treatment device according to claim 1,
wherein at least two bio-liquid treatment modules (12, 14, 16) comprise at least one bio-liquid inlet (12a, 14a, 16a) and at least one bio-liquid outlet (12b, 14b, 16b), respectively, at least two of the bio-liquid treatment modules being connected in series with respect to the flow of bio-liquid (BL) defined by said bio-liquid inlets and said bio-liquid outlets.

3. Bio-liquid treatment device according to claim 1 or 2,
wherein at least one bio-liquid treatment module (12, 14, 16) comprises a bio-liquid compartment (12e, 14e, 16e) and a working liquid compartment (12f, 14f, 16f) , said working liquid compartment comprising a working liquid inlet (12c, 14c, 16c) and a working liquid outlet (12d, 14d, 16d), and said bio-liquid compartment and said working liquid compartment being separated from each other by a separating medium (12g, 14g, 16g) separating the bio-liquid (BL) from the working liquid (WL) but allowing the transfer of predetermined substances.

4. Bio-liquid treatment device according to claim 3,
wherein said separating medium (12g, 14g, 16g) is selected from a membrane, activated carbon, and absorption/adsorption particles and/or ion exchange media and/or adsorption membrane.

5. Bio-liquid treatment device according to claim 4,
wherein the membrane is formed by the wall material of a plurality of hollow fibers (20).

6. Bio-liquid treatment device according to claim 5,
wherein the fiber material is at least one material selected from a group consisting of polysulphone, polymethyl methacrylate, polyamide, polyethersulphone, polyetherimide, cuprophane, and hemophane.

7. Bio-liquid treatment device according to any of claims 3 to 6,
wherein in the working liquid compartment (74f) of at least one bio-liquid treatment module (74) there is provided a bio-liquid treatment medium (74h), e.g. activated carbon and/or absorption/adsorption particles.

8. Bio-liquid treatment device according to any of claims 3 to 7,
wherein the working liquid inlet (74c) of at least one bio-liquid treatment module (74) is closed.

9. Bio-liquid treatment device according to any of claims 3 to 8,
wherein the working liquid compartments (12f, 14f, 16f) are connected in series with respect to the flow of working liquid (WL), the working liquid (WL) flowing in counter-flow with respect to the flow of bio-liquid (BL).

10. Bio-liquid treatment device according to claim 9,
wherein an intermediate bio-liquid treatment module (14) of the series of bio-liquid treatment modules (12, 14, 16) has a higher flow resistance than a preceding bio-liquid treatment module (12) and a succeeding bio-liquid treatment module (16).

11. Bio-liquid treatment device according to claim 9 or 10,
wherein a final bio-liquid treatment module (16) of the series of bio-liquid treatment modules (12, 14, 16) has a lower flow resistance than any of the preceding bio-liquid treatment modules (12, 14).

12. Bio-liquid treatment device according to any of claims 9 to 11,
wherein overall length of the hollow fibers (20) in the preceding module (12), the intermediate module (14) and the succeeding module (16) amounts to between about 50 mm and about 230 mm.

13. Bio-liquid treatment device according to any of claims 1 to 12,
wherein at least one bio-liquid treatment module (12) has a housing (26) separate from the housing (28) of the other bio-liquid treatment modules (14, 16).

14. Bio-liquid treatment device according to any of claims 1 to 13,
wherein at least two bio-liquid treatment modules (14, 16) share a common housing (28).

15. Bio-liquid treatment device according to claim 13 or 14,
wherein the housing (26, 42, 44) has an overall longitudinal shape, the outer contour of which is substantially constant over the entire length of the housing.

16. Bio-liquid treatment device according to any of claims 13 to 15,
wherein the outer surface of the cross section of the housing (26, 42, 44) has a substantially polygonal or at least partially curved contour.

17. Bio-liquid treatment device according to any of claims 13 to 16,
wherein the connection (36) between at least two housings (26, 42, 44) is an interlocking connection.

18. Bio-liquid treatment device according to any of claims 13 to 17,
wherein the housing (26, 42, 44) comprises at least one of a male connecting structure (26a, 42a, 44a) and a female connecting structure (26b, 42b, 44b).

19. Bio-liquid treatment device according to claim 18,
wherein at least one pair of mating connecting structures (26a-26b, 42a-42b, 44a-44b) is formed as a slide-and-lock-connection.

20. Bio-liquid treatment device according to claim 18 or 19,
wherein the male connecting structure (26a, 42a, 44a) is formed by a dovetail-like projection.

21. Bio-liquid treatment device according to any of claims 18 to 20,
wherein the female connecting structure (26b, 42b, 44b) is formed by dovetail-like recess.

22. Bio-liquid treatment device according to any of claims 17 to 21,
wherein at least one connecting structure (26a, 42a, 44a, 26b, 42b, 44b) has at least one through-hole (38) leading to the interior of the housing (26, 42, 44).

23. Bio-liquid treatment device according to any of claims 13 to 22,
wherein a ring chamber (41) is provided in at least one longitudinal end section of the housing (26).

24. Bio-liquid treatment device according to claim 23,
wherein the dimension of the ring chamber (41) taken in the longitudinal direction (L) of the housing (26) amounts to between about 0.1 mm and about 3.0 mm.

25. Bio-liquid treatment device according to claim 23 or 24,
wherein the inner diameter of the housing (26) is increased in at least one longitudinal end section.

26. Bio-liquid treatment device according to any of claims 23 to 25,
wherein the cross-section of the envelope surface of the bundle of hollow fibers (20) is reduced in at least one longitudinal end section of the housing (26).
